# EUROPEAN PATENT SPECIFICATION

(11) **EP 4 311 524 B1**
(45) Date of publication and mention of the grant of the patent: **11.02.2026**
(21) Application number: 23188536.9
(22) Date of filing: 28.07.2023
(51) Int. Cl.: A61F 2/38

(54) **SYSTEM FOR MAKING COMPENSATION ELEMENTS**
SYSTEM ZUR HERSTELLUNG VON KOMPENSATIONSELEMENTEN
SYSTÈME DE FABRICATION D'ÉLÉMENTS DE COMPENSATION

(30) Priority: 28.07.2022 IT 202200015954
(43) Date of publication of application: 31.01.2024
(73) Proprietor: Balato, Giovanni, 81030 Lusciano (CE) (IT); Balato, Marco, 81031 Aversa (CE) (IT)
(72) Inventor: BALATO, Giovanni, 81030 Lusciano (CE) (IT); BALATO, Marco, 81031 Aversa (CE) (IT); PETRARCA, Carlo, 81100 Caserta (CE) (IT); LENZI, Marco, 28921 Verbania (VB) (IT); D'AMATO, Michele, 29121 Piacenza (PC) (IT); FESTA, Enrico, 80013 Napoli (NA) (IT)
(74) Representative: Santi, Filippo

(56) References cited:
- WO-A1-2016/183446
- US-A1- 2001 039 455
- US-B2- 8 382 849

## Description

The present invention relates to a system for preparing and producing compensatory elements, in particular symmetrical and/or asymmetrical femoral and tibial augments and/or cones made of bone cement for an articulated spacer suitable for being temporarily implanted at the joint between the tibia and femur. Furthermore, the present invention relates to a method for preparing customised compensatory elements based on the patient's anatomic needs.

Infection represents a terrible complication of joint replacement surgery.

The optimal result of treatment of septic failure of a prosthetic joint implant is represented by the eradication of the infection and reimplantation of a non-painful, well-functioning joint prosthesis. Various therapeutic options are available.

In cases where the infection is delayed (with onset between 3 months and 2 years after implantation) or is caused by highly virulent germs, with compromise of periprosthetic soft tissues, the surgical treatment provides for removal of the implant and the positioning of an antibiotic-loaded cement spacer with subsequent reimplantation of a prosthesis once recovery has been achieved (two-stage technique).

This technique is by now considered the gold standard for managing periprosthetic knee infections, with a percentage of success ranging from 73 to 100%. The choice of the type of spacer to be used is fundamental and must meet criteria that are well defined by the scientific literature. In particular, the rationale for selecting a spacer of a mobile or static type is based on various factors, including the type of germ, state of the skin and soft tissues, whether or not osteomyelitis is present and the degree of compromise of the patient. In detail, if the germ is not very virulent and has a good sensitivity profile, the patient is in good overall conditions and the periprosthetic soft tissues are in a good state, the use of a spacer of a mobile type, such as the one described by Hofmann et al. in 1995, could be indicated. Mobile spacers, unlike fixed ones, enable flexion and extension of the knee during the period that follows removal of the prosthesis, reaching values even greater than 90° of flexion, thus avoiding rigidity and osteopenia without compromising the eradication of the infection and making the second stage of surgery certainly easier for the surgeon. However, even articulated spacers can give rise to complications. The excessive load and the movement to which they are sometimes subjected can cause the breakage and even mobilisation thereof, with major reactive synovitis. Not to mention the rigidity during flexion that is mainly observed in preformed mobile spacers as opposed to the spacers according to Hofmann. With regard to contraindications, we can maintain that articulated spacers should not be used in infections associated with a destruction of the extensor apparatus, or severe ligament instability due to incompetent or absent collaterals, because complications such as actual knee dislocations or dehiscence of the surgical wound can be observed.

Generally speaking, the articulated spacers known in the literature (Freeman MG et al. "Functional advantage of articulating versus static spacers in 2-stage revision for total knee arthroplasty infection". J Arthroplasty. 2007 Dec;22(8):1116-15 21; Vasso M, et al. "Articulated spacer provides long-term knee improvement after two-stage reimplantation. Knee Surg Sports Traumatol Arthrosc". 2016 Oct;24(10):3100-3105) have the geometric shape of the prosthetic implant and can be either premade or prepared in the operating room with moulds made of plastic material and not only, into which bone cement is introduced after a manual or mechanical mixing step.

Example of system for producing a compensatory element are shown in US 2001/039455 A1 and in US 8 382 849 B2.

During the operation, the surgeon proceeds to choose the implant with the aim of
i) correcting deformities in the spatial planes,
ii) restoring the joint line and
iii) filling the space in extension and flexion so as to ensure the stability of the joint.

The surgeon often finds him- or herself having to manage both femoral-tibial and femoral-patellar instability, malalignments on the front plane, bone defects, both segmental and cavitary (involving the joint surfaces, metaphyses and diaphyses), and defects in the positioning of the components in the horizontal plane (rotational defects) without the possibility of dedicated instruments that might speed up and ensure the resolution of such problems.

It is an aim of the present invention to overcome the above-mentioned problems, in whole or in part, and to provide an instrument intended to correct bone defects and ensure a better fixation of the spacer, by using additional components: cones, symmetrical (full block) and asymmetrical femoral and tibial augments.

Furthermore, one aim of the present invention is to provide a system that makes it possible to produce compensatory elements for patients with different physiques.

The system according to the present invention imports the concept of reconstructive prosthesis revision surgery, starting from the instruments typical of this surgery (cones and augments) and adapting them to a spacer which, by definition, is temporary and not customised. These devices enable a surgeon to suitably manage various clinical situations, including both cavitary and segmental bone defects. Augments maintain a close interface both with the spacer and with the bone and the surrounding tissues of the patient, allowing the surgeon to implant such devices in a variety of positions, before or after positioning the implant, in order to adapt to different bone anatomies and the knee spacer. Furthermore, a cone provides the surgeon with the possibility of compensating for cavitary bone defects, both femoral and tibial, while at the same time providing a more stable and lasting fixation of the spacer.

The invention is defined in the claims. A specific object of the present invention is a system for producing compensatory elements to be used intraoperatively to manage the implantation of a temporary spacer in the presence of segmental and/or cavitary bone defects of the tibia and/or femur, said system being characterised in that it comprises
at least one cavity configured to mould a symmetrical tibial augment, said cavity having the shape of a half-disk, and
at least one cavity configured to mould a symmetrical femoral augment, said cavity having the shape of a parallelepiped, and/or
at least one cavity configured to mould an asymmetrical augment, said cavity being U-shaped, and
at least one cavity configured to mould a tibial cone, said cavity having the shape of a truncated cone with an upper base larger than a lower base, and
at least one cavity configured to mould a femoral cone, said cavity having the shape of an elliptical truncated cone with an upper base larger than a lower base.

According to the invention, said system can comprise a tibial kit and a femoral kit,
said tibial kit comprising said at least one cavity configured to mould a symmetrical tibial augment, said at least one cavity configured to mould an asymmetrical augment and said at least one cavity configured to mould a tibial cone, and
said femoral kit comprising said at least one cavity configured to mould a symmetrical femoral augment and said at least one cavity configured to mould a femoral cone.

In particular, according to the invention, said at least one cavity configured to mould a symmetrical tibial augment and said at least one cavity configured to mould a symmetrical femoral augment can have a thickness comprised between 2mm and 20mm.

Likewise according to the invention, said at least one cavity configured to mould said asymmetrical augment can have a thickness selected from 3mm, 5mm, and 10mm, a length selected from 33.4mm and 47mm and a width selected from 48mm and 61mm.

In particular, according to the invention, said at least one cavity configured to mould said tibial cone can have a height A₁ equal to 25mm, a smaller diameter C₁ selected from 16.08mm and 17.98mm and a larger diameter D₁ selected from 27.12mm and 28.83mm.

Moreover, according to the invention, said at least one cavity configured to mould said femoral cone can have a maximum length C₂ selected from 25.5mm and 27.42mm and a maximum width D₂ selected from 22.58mm and 24.28mm.

Furthermore, according to the invention, the outer surface of said at least one cavity configured to mould a tibial cone and of said at least one cavity configured to mould a femoral cone can comprise a portion with steps.

A further object of the present invention is a method for producing at least one compensatory element by means of a system, comprising the following steps:
- providing a system for producing compensatory elements according to any of claims 1-7,
- selecting a cavity based on the shape and/or size aspects of the compensatory element to be produced;
- introducing bone cement into said cavity;
- extracting the compensatory element from said cavity when the bone cement has hardened.

According to the invention, said method can further comprise the following step
- pressing the bone cement introduced into said system

The present invention will now be described, by way of non-limiting illustration, according to a preferred embodiment, with particular reference to the figures in the appended drawings, wherein:
- figure 1a shows a front perspective view of a system according to the present invention,
- figure 1b shows a rear perspective view of the system in figure 1a,
- figure 2a shows a perspective view of a symmetrical tibial augment obtained by means of the system in figure 1a,
- figure 2b shows a view of the symmetrical tibial augment during a step of application in the area to be treated,
- figure 3a shows a perspective view of a symmetrical femoral augment obtained by means of the system in figure 1a,
- figure 3b shows a view of the symmetrical femoral augment during a step of application in the area to be treated,
- figures 4a-4e show an asymmetrical augment obtained by means of the system in figure 1a,
- figure 5 shows a view of the asymmetrical augment during a step of application in the area to be treated,
- figure 6a shows a perspective view of a tibial cone obtained by means of the system in figure 1a,
- figure 6b shows a side view of the tibial cone in figure 6a,
- figure 6c shows a top view of the tibial cone in figure 6a,
- figure 6d shows a view of the tibial cone during a step of application in the area to be treated,
- figure 7a shows a perspective view of a femoral cone obtained by means of the system in figure 1a,
- figure 7b shows a side view of the femoral cone in figure 7a,
- figure 7c shows a top view of the femoral cone in figure 7a, and
- figure 7d shows a view of the femoral cone during a step of application in the area to be treated.

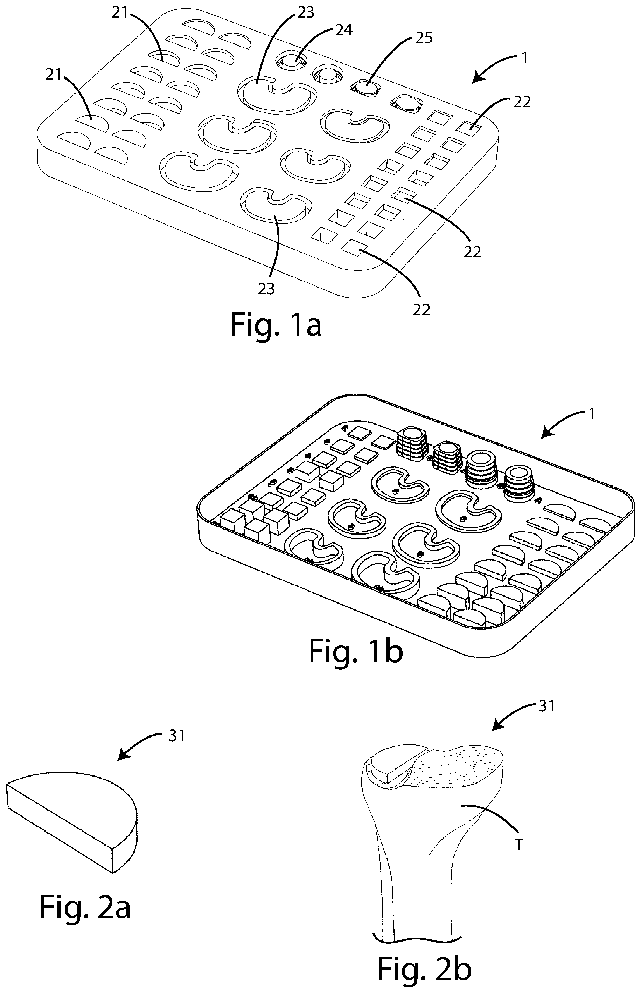

Making reference to figures 1a-1b, the number 1 denotes a system for preparing compensatory elements according to the present invention.

In particular, said system 1 comprises a plurality of cavities, each cavity having a shape complementary to that of a respective compensatory element, such as an augment. Said compensatory elements are adapted to be positioned in an area to be treated of the patient's tibia T and/or femur F. In particular, in orthopaedic surgery, an augment is a piece of material used to reconstruct or fill a missing or damaged bone part. Augments are designed to provide structural support and improve bone integrity.

More particularly, said system 1 comprises at least one cavity 21 configured to mould a symmetrical tibial augment 31 aimed at managing tibial epiphyseal segmental bone defects.

Making reference to figure 2a, said symmetrical tibial augment 31 comprises a sphere portion with a thickness ranging from 2mm to 20 mm, with a discretised, non-uniform pitch. In particular, again making reference to figure 2a, said symmetrical tibial augment 31 comprises a closed line that borders a contact surface, said contact surface being represented by a portion of a circumference. In the embodiment shown in figure 2a, said portion of a circumference has a radius equal to 16mm and diameter equal to 32 mm.

In the embodiment shown in figure 1a, the system 1 according to the present invention comprises at least eight cavities 21 configured to mould said symmetrical tibial augment 31. In particular, said eight cavities 21 have a thickness selected from 2mm, 3mm, 4mm, 5mm, 6mm, 10mm, 15mm and 20mm in order to enable management of the totality of tibial epiphyseal segmental bone defects.

Moreover, said system 1 comprises at least one cavity 22 configured to mould a symmetrical femoral augment 32. The symmetrical femoral augments 32, comprise a square contact surface, for example with a side equal to 16mm and variable thickness, for example from 2mm to 20mm with a discretised, non-uniform pitch.

In the embodiment shown in figure 1a, the system 1 according to the present invention comprises eight cavities 22 configured to mould said symmetrical femoral augment 32. In particular, said eight cavities 22 have a thickness selected from 2mm, 3mm, 4mm, 5mm, 6mm, 10mm, 15mm and 20mm in order to enable management of the totality of tibial epiphyseal segmental bone defects.

Moreover, said system 1 comprises at least one cavity 23 configured to mould an asymmetrical augment 33. The asymmetrical augments fulfil the different needs for augmentation of the tibial epiphysis, since very often the only supporting surface is the circumferential cortical one, as the spongy one is completely lacking.

The property of asymmetry is obtained by considering as uniform the thickness D along the whole contact surface between said augment and the tibia.

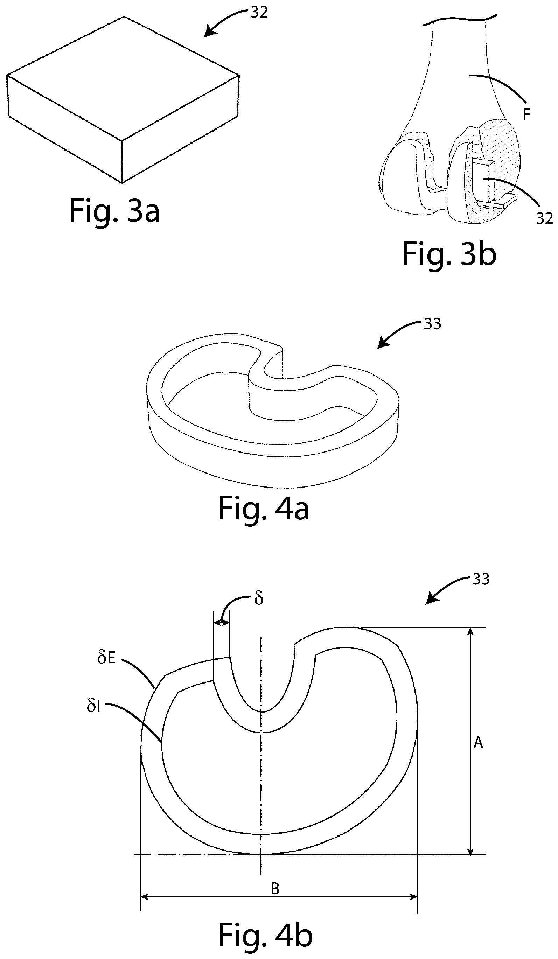

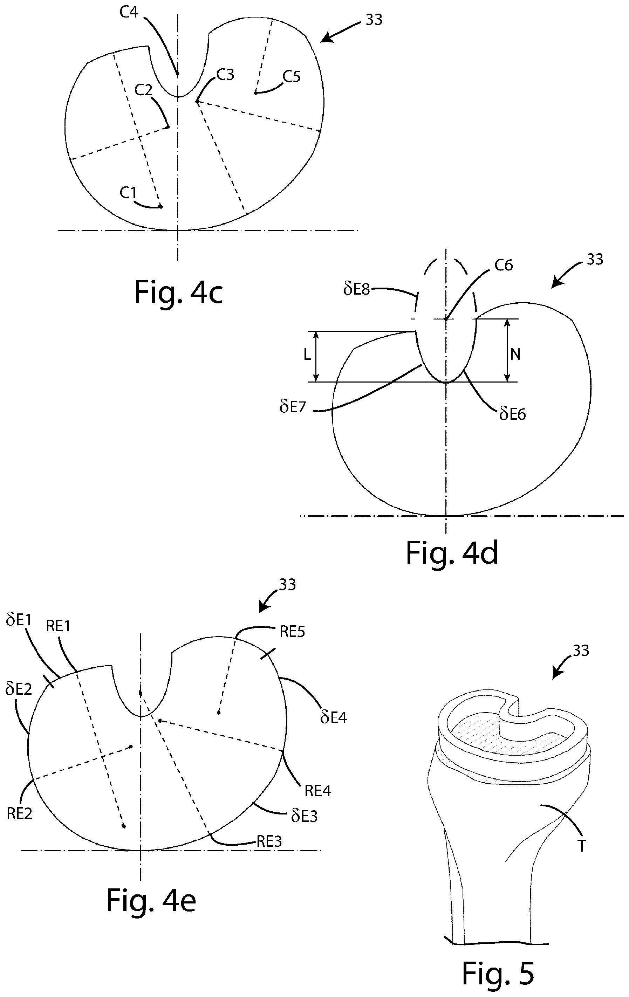

Making reference to figures 4b-4e, said asymmetrical augment 33 comprises a contact surface obtained as a difference between a surface bordered by an external closed line δE and a surface bordered by an internal closed line δl. Both of these closed lines are obtained by joining seven open lines, that is, five circumferential arcs and two elliptical arcs, respectively denoted as δE1, δE2, δE3, δE4, δE5, δE6 and δE7 and δI1, δI2, δI3, δI4, δI5, δI6 and δI7.

The properties that characterise the homologous circumferential arcs (δEj and δIj with j=1, 2,..., 5) are the following:
1) CEj = CIj = Cj with j=1, 2,...,5 (same centre);
2) REj-RIj = δ with j=1, 2, ..., 5 and δ=5mm.

Where CEj (CIj) indicates the centre of the j-th external (internal) circumferential arc and REj (RIj) indicates the radius of the j-th external (internal) circumferential arc.

Finally, the open lines, both internal (δI6 and δI7) and external (δE6 and δE7), represented by elliptical arcs, enjoy the following properties:
1) CEj = CIj = Cj with j=6 (same centre);
2) NEj-NIj= δ with j=6, 7 and δ=5mm (semi-major axis);
3) LEj-LIj= S with j=6, 7 and δ=5mm (semi-minor axis).

Where NEj (NIj) indicates the extent of the external (internal) semi-major axis and LEj (LIj) indicates the extent of the external (internal) semi-minor axis.

The result is a particular geometry characterised by a high degree of matching.

In the embodiment shown in figure 1a, the system 1 according to the present invention comprises three cavities 23 configured to mould said asymmetrical augment 33. In particular, said three cavities 23 have a thickness selected from 3mm, 5mm, and 10mm, a length selected from 33.4mm and 47mm and a width selected from 48mm and 61mm in order to enable adequate coupling with the tibial bone component in the event that there is a total absence of spongy bone.

Moreover, said system 1 comprises at least one cavity 24 configured to mould a tibial cone 34 and at least one cavity 25 configured to mould a femoral cone 35. The creation of said cones 34, 35 is aimed at compensating for cavitary bone defects involving the femoral and tibial metaphysis which can result from a severe infection process or a difficult extraction of an infected prosthesis.

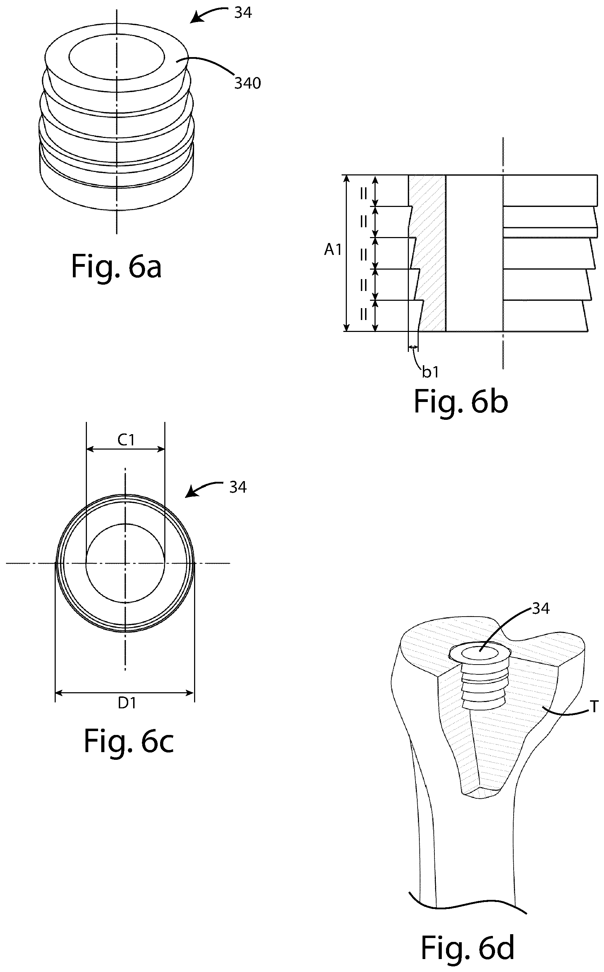

Making reference to figures 6a-6d, said tibial cones are obtained by joining five identical truncated cones 340 with a height II, larger diameter D₁ and smaller diameter C₁. The entire geometry, obtained by making the lower base coincide with the upper base of the truncated cones placed in sequence, shows an overall height A₁ equal to 5*II (A₁=5*II). The dimensions of the cavity 24 are those of a cylinder with a height and diameter coinciding, respectively, with the height A₁ and with the smaller diameter C₁ of the tibial cone.

In the embodiment shown in figure 1a, the system 1 according to the present invention comprises at least one cavity 24 configured to mould said tibial cone 34. In particular, said at least one cavity 24 has a height A₁ equal to 25mm, the smaller diameter C₁ being selected from 16.08mm and 17.98mm and the larger diameter D₁ selected from 27.12mm and 28.83mm.

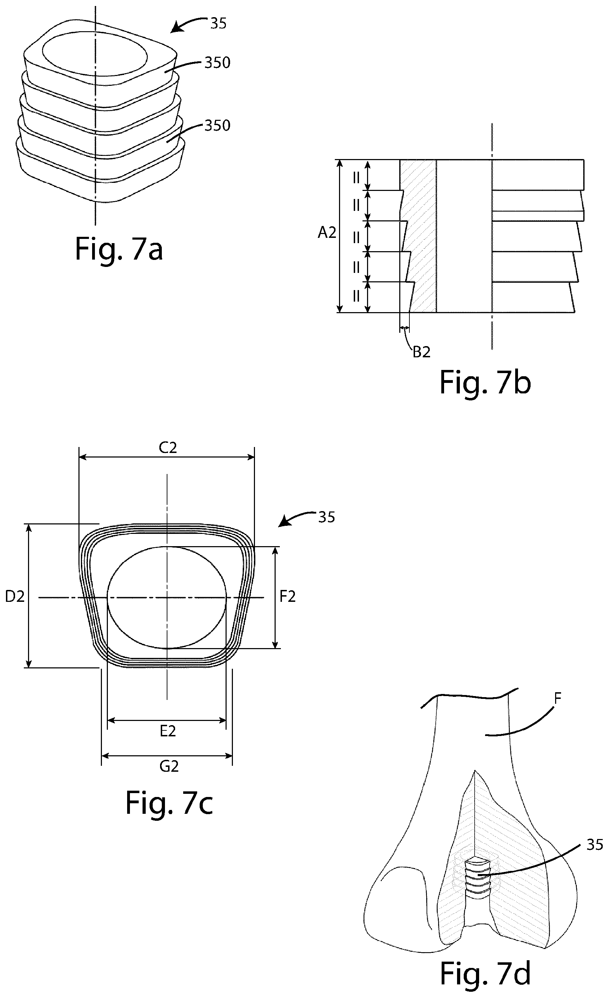

Making reference to figures 7a-7d, said femoral cones 35 are obtained by joining five identical hollow truncated pyramids 350 with a trapezoidal base, rounded corners and a height II, preferably equal to 5mm. The entire geometry shows an overall height A₂=25 mm, equal to 5*II (A₂=5*II). The dimensions of the cavity are those of an elliptical cylinder with a height equal to A₂, major axis E₂, preferably E₂=23.55mm, and minor axis F₂, preferably F₂=19.5mm.

In the embodiment shown in figure 1a, the system 1 according to the present invention comprises at least one cavity 25 configured to mould said femoral cone 35. In particular, said at least one cavity 25 has a maximum length C₂ selected from 25.5mm and 27.42mm and a maximum width D₂ selected from 22.58mm and 24.28mm.

Described below are the ways in which it is possible to create said system for producing symmetrical and asymmetrical augments and femoral and tibial cones with bone cement, to be used intraoperatively to manage:
i) segmental bone defects of the tibia and/or femur, and
ii) cavitary defects of the tibia and/or femur.

Said system can be obtained with the aid of a 3D printer. In particular, moulds are created which serve as positives, from which the process of producing symmetrical and asymmetrical augments and cones originates. The workflow started from design development by means of open-source commercial CAD software, the final product of which was a file in STL format. Positive moulds were thus modelled for square-shaped femoral augments with a variable thickness (from 2mm to 20mm), symmetrical augments with a half-moon shape and variable thickness (from 2mm to 20mm), tibial cones in two sizes (small and large), femoral cones in two sizes (small and large) and asymmetrical augments conforming to the anatomy of the tibial plateau, with a variable thickness (from 3mm to 10mm) and in two sizes (small and large). Once the virtual models were obtained, they were subjected to a slicing procedure using commercial software (for example Cura 4.11^{©} Ultimaker B.V. Stationsplein 32 3511 ED, Utrecht), for setting the printing process using the highest possible resolution of the layers and avoiding the use of print media as much as possible. We thus obtained a gcode file optimised for the 3D printers at our disposal (FDM Creality Ender 3 pro, 18F, JinXiuHongDu Building, Meilong Blvd., Longhua Dist., Shenzhen, 10 China 518131) with a 0.1-0.4mm nozzle and a theoretical printing precision of ±0.1mm. The virtual model in the gcode format is imported into the printer on which pre-printing checks were previously carried out. The current strategy provides for the printing of a positive container-model using standard PLA at the maximum available layer definition (0.1mm), with a standard infill set at 30%, an extrusion temperature of 180° and a bed temperature of 50°C for moulds requiring greater adhesion to the print bed. Once printing is completed, thorough checks are performed to verify correspondence with the set dimensions, with a maximum tolerance of ±0.1mm, and the surfaces, prepared with ultrafine sandpaper (grit <400), will serve as a mould for casting of the negative mould material.

The need to have an autoclavable material, i.e. one that can withstand unchanged the preliminary decontamination/disinfection and sterilisation processes in an autoclave (at least 134 °C at a pressure of 2 bars for about 30 min), precluded the use of thermolabile materials such as the filaments for FDM printers. The choice fell on silicone rubber for casting (shore 50). The process consists in the casting of the material, to which a catalyst is added, in the rigid PLA mould, which is subsequently positioned in a container that assures negative pressure, with the aim of removing any air bubbles that may have formed; otherwise, the mould produced would be discarded. The material requires 24h for complete catalysis, after which it can be removed from the positive PLA mould, checked, decontaminated, sterilised and stored in a double sterile container. In a sterile environment, the negative silicone rubber mould is positioned on a surface. The PMMA cement, to which the combination of antibiotics suitable for the patient, based on the antibiogram profile (if available), is added, is prepared separately under sterile conditions.

Furthermore, with the objective of reaching mass production, said system can be a stainless steel mould produced with traditional swarf removal machines (3-axis CNC milling machine). The product obtained is easily sterilisable compared to the one made of PLA. For the production of the (negative) mould, the choice fell on platinum-cured silicone rubber whose characteristics are: impermeability to external contamination once catalysed, catalysation also under vacuum, odourless, process with controllable catalysation times, and sterilisable in an autoclave, by ultrasound and with the ethylene oxide gas method. In other respects, the production process is nearly identical to what has been described for the system obtained with 3D printing.

Furthermore, the present invention relates to a method for producing a compensatory element. The method envisages providing a system produced according to the embodiment shown in figure 1a. Therefore, the cavities to be filled are selected based on the characteristics of the patient's physique, i.e. based on the dimensions of the bone portions in which the compensatory element intervenes. In addition, the amount of cement introduced into each cavity defines the longitudinal dimensions of the compensatory element. Subsequently, the method can envisage a pressing step and a consequent step of solidification of the bone cement, so as to obtain the compensatory element.

The present invention has been described by way of non-limiting illustration, according to the preferred embodiments thereof, but it is to be understood that variations and/or modifications may be introduced by persons skilled in the art without going beyond the relevant scope of protection, as defined by the appended claims.

## Claims

1. A system (1) for producing compensatory elements (31, 32, 33, 34, 35) to be used intraoperatively to manage the implantation of a temporary spacer in the presence of segmental and/or cavitary bone defects of the tibia (T) and/or femur (F), said system (1) comprising at least one cavity (24) configured to mould a tibial cone (34), said cavity (24) having the shape of a truncated cone with an upper base larger than a lower base, the system being **characterised in that** it further comprises at least one cavity (21) configured to mould a symmetrical tibial augment (31), said cavity (21) having the shape of a half-disk, and
at least one cavity (22) configured to mould a symmetrical femoral augment (32), said cavity (22) having the shape of a parallelepiped, and/or
at least one cavity (23) configured to mould an asymmetrical augment (33), said cavity (23) being U-shaped, and
at least one cavity (25) configured to mould a femoral cone (35), said cavity (35) having the shape of an elliptical truncated cone with an upper base larger than a lower base.

2. The system (1) according to the preceding claim, **characterised in that** it comprises a tibial kit and a femoral kit,
said tibial kit comprising said at least one cavity (21) configured to mould a symmetrical tibial augment (31), said at least one cavity (23) configured to mould an asymmetrical augment (33) and said at least one cavity (24) configured to mould a tibial cone (34), and
said femoral kit comprising said at least one cavity (22) configured to mould a symmetrical femoral augment (32) and said at least one cavity (25) configured to mould a femoral cone (35).

3. The system (1) according to any one of the preceding claims, **characterised in that** said at least one cavity (21) configured to mould a symmetrical tibial augment (31) and said at least one cavity (22) configured to mould a symmetrical femoral augment (32) have a thickness comprised between 2mm and 20mm.

4. The system (1) according to any one of the preceding claims, **characterised in that** said at least one cavity (23) configured to mould said asymmetrical augment (33) has a thickness selected from 3mm, 5mm, and 10mm, a length selected from 33,4mm and 47mm and a width selected from 48mm and 61mm.

5. The system (1) according to any one of the preceding claims, **characterised in that** said at least one cavity (24) configured to mould said tibial cone (34) has a height A₁ equal to 25mm, a smaller diameter C₁ selected from 16.08mm and 17.98mm and a larger diameter D₁ selected from 27.12mm and 28.83mm.

6. The system (1) according to any one of the preceding claims, **characterised in that** said at least one cavity (25) configured to mould said femoral cone (35) has a maximum length C₂ selected from 25.5mm and 27.42mm and a maximum width D₂ selected from 22.58mm and 24.28mm.

7. The system (1) according to any one of the preceding claims, **characterised in that** the outer surface of said at least one cavity (24) configured to mould a tibial cone (34) and of said at least one cavity (25) configured to mould a femoral cone (35) comprises a portion with steps.

8. A method for producing at least one compensatory element (31, 32, 33, 34, 35) by means of a system (1) according to one of claims 1-7, comprising the following steps:
providing a system according to any of claims 1-7;
- selecting a cavity (21, 22, 23, 24, 25) based on the shape and/or size aspects of the compensatory element to be produced;
- introducing bone cement into said cavity (21, 22, 23, 24, 25); and
- extracting the compensatory element from said cavity (21, 22, 23, 24, 25) when the bone cement has hardened.

9. The method according to the preceding claim, **characterised in that** it further comprises the following step
- pressing the bone cement introduced into said system (1).

## Patentansprüche

1. System (1) zur Herstellung von Kompensationselementen (31, 32, 33, 34, 35), die intraoperativ verwendet werden sollen, um die Implantation eines temporären Abstandshalters bei segmentalen und/oder kavitären Knochendefekten des Schienbeins (T) und/oder des Oberschenkelknochens (F) zu steuern, wobei das System (1) mindestens einen Hohlraum (24) umfasst, der so konfiguriert ist, dass er einen Tibiakonus (34) formt, wobei der Hohlraum (24) die Form eines Kegelstumpfes hat, dessen obere Basis größer ist als die untere Basis, wobei das System **dadurch gekennzeichnet ist, dass** es außerdem mindestens einen Hohlraum (21) umfasst, der so konfiguriert ist, dass er ein symmetrisches tibiales Augment (31) formt, wobei der Hohlraum (21) die Form einer halben Scheibe hat, und
mindestens einen Hohlraum (22), der so gestaltet ist, dass er eine symmetrische Oberschenkelvergrößerung (32) formt, wobei der Hohlraum (22) die Form eines Parallelepipeds hat, und/oder
mindestens einen Hohlraum (23), der so gestaltet ist,
dass er einen asymmetrischen Fortsatz (33) formt, wobei der Hohlraum (23) U-förmig ist, und
mindestens einen Hohlraum (25), der so gestaltet ist, dass er einen Femurkonus (35) formt, wobei der Hohlraum (35) die Form eines elliptischen Kegelstumpfes hat, dessen obere Basis größer ist als die untere Basis.

2. System (1) nach dem vorhergehenden Anspruch, **dadurch gekennzeichnet, dass** es ein Tibia-Kit und ein Femur-Kit umfasst,
der Tibia-Bausatz umfasst den mindestens einen Hohlraum (21), der so konfiguriert ist, dass er ein symmetrisches Tibia-Augment (31) formt, den mindestens einen Hohlraum (23), der so konfiguriert ist, dass er ein asymmetrisches Augment (33) formt, und den mindestens einen Hohlraum (24), der so konfiguriert ist, dass er einen Tibia-Konus (34) formt, und
wobei der Oberschenkelsatz den mindestens einen Hohlraum (22), der zum Formen einer symmetrischen Oberschenkelerweiterung (32) konfiguriert ist, und den mindestens einen Hohlraum (25), der zum Formen eines Oberschenkelkonus (35) konfiguriert ist, umfasst.

3. System (1) nach einem der vorhergehenden Ansprüche, **dadurch gekennzeichnet, dass** der mindestens eine Hohlraum (21), der zum Formen eines symmetrischen tibialen Augmentats (31) konfiguriert ist, und der mindestens eine Hohlraum (22), der zum Formen eines symmetrischen femoralen Augmentats (32) konfiguriert ist, eine Dicke zwischen 2mm und 20mm aufweisen.

4. System (1) nach einem der vorhergehenden Ansprüche, **dadurch gekennzeichnet, dass** der mindestens eine Hohlraum (23), der zum Formen der asymmetrischen Vergrößerung (33) konfiguriert ist, eine Dicke aufweist, die aus 3mm, 5mm und 10mm ausgewählt ist,
eine Länge, die zwischen 33,4mm und 47mm ausgewählt wird, und eine Breite, die zwischen 48mm und 61mm ausgewählt wird.

5. System (1) nach einem der vorhergehenden Ansprüche, **dadurch gekennzeichnet, dass** der mindestens eine Hohlraum (24), der zum Formen des Tibiakonus (34) konfiguriert ist, eine Höhe A₁ gleich 25mm, einen kleineren Durchmesser C₁, ausgewählt aus 16,08mm und 17,98mm, und einen größeren Durchmesser D₁, ausgewählt aus 27,12mm und 28,83mm, aufweist.

6. System (1) nach einem der vorhergehenden Ansprüche, **dadurch gekennzeichnet, dass** der mindestens eine Hohlraum (25), der zum Formen des Femurkonus (35) konfiguriert ist, eine maximale Länge C₂, ausgewählt aus 25,5mm und 27,42mm, und eine maximale Breite D₂, ausgewählt aus 22,58mm und 24,28mm, aufweist.

7. System (1) nach einem der vorhergehenden Ansprüche, **dadurch gekennzeichnet, dass** die Außenfläche des mindestens einen Hohlraums (24), der zum Formen eines Tibiakonus (34) konfiguriert ist, und des mindestens einen Hohlraums (25), der zum Formen eines Femurkonus (35) konfiguriert ist, einen Abschnitt mit Stufen umfasst.

8. Verfahren zur Herstellung mindestens eines Ausgleichselements (31, 32, 33, 34, 35) mittels eines Systems (1) nach einem der Ansprüche 1-7, umfassend die folgenden Schritte:
Bereitstellen eines Systems gemäß einem der Ansprüche 1-7;
- Auswahl einer Kavität (21, 22, 23, 24, 25) auf der Grundlage der Form- und/oder Größenaspekte des herzustellenden Kompensationselements;
- Einbringen von Knochenzement in den genannten Hohlraum (21, 22, 23, 24, 25); und
- Herausziehen des Ausgleichselements aus dem Hohlraum (21, 22, 23, 24, 25), wenn der Knochenzement ausgehärtet ist.

9. Verfahren gemäß dem vorstehenden Anspruch, **dadurch gekennzeichnet, dass** es ferner den folgenden Schritt umfasst
- Verpressen des in das System (1) eingebrachten Knochenzements.

## Revendications

1. Système (1) de production d'éléments compensatoires (31, 32, 33, 34, 35) à utiliser en peropératoire pour gérer l'implantation d'un écarteur temporaire en présence de défauts osseux segmentaires et/ou cavitaires du tibia (T) et/ou du fémur (F), ledit système (1) comprenant au moins une cavité (24) configurée pour mouler un cône fémoral (34), ladite cavité (24) ayant la forme d'un tronc de cône avec une base supérieure plus grande que la base inférieure, le système étant **caractérisé en ce qu'**il comprend en outre au moins une cavité (21) configurée pour mouler une augmentation fémorale symétrique (31), ladite cavité (21) ayant la forme d'un demi-disque, et
au moins une cavité (22) configurée pour mouler une augmentation fémorale symétrique (32), ladite cavité (22) ayant la forme d'un parallélépipède, et/ou au moins une cavité (23) configurée pour mouler une augmentation asymétrique (33), ladite cavité (23) étant en forme de U, et
au moins une cavité (25) configurée pour mouler un cône fémoral (35), ladite cavité (35) ayant la forme d'un cône tronqué elliptique dont la base supérieure est plus grande que la base inférieure.

2. Système (1) selon la revendication précédente, **caractérisé en ce qu'**il comprend un kit fémoral et un kit fémoral,
ledit kit fémoral comprenant ladite au moins une cavité (21) configurée pour mouler une augmentation fémorale symétrique (31), ladite au moins une cavité (23) configurée pour mouler une augmentation asymétrique (33) et ladite au moins une cavité (24) configurée pour mouler un cône fémoral (34), et
ledit kit fémoral comprenant ladite au moins une cavité (22) configurée pour mouler une augmentation fémorale symétrique (32) et ladite au moins une cavité (25) configurée pour mouler un cône fémoral (35).

3. Système (1) selon l'une quelconque des revendications précédentes, **caractérisé en ce que** ladite au moins une cavité (21) configurée pour mouler une augmentation fémorale symétrique (31) et ladite au moins une cavité (22) configurée pour mouler une augmentation fémorale symétrique (32) ont une épaisseur comprise entre 2mm et 20mm.

4. Système (1) selon l'une quelconque des revendications précédentes, **caractérisé en ce que** ladite au moins une cavité (23) configurée pour mouler ladite augmentation asymétrique (33) a une épaisseur choisie entre 3mm, 5mm et 10mm,
une longueur choisie entre 33,4 mm et 47 mm et une largeur choisie entre 48 mm et 61 mm.

5. Système (1) selon l'une quelconque des revendications précédentes, **caractérisé en ce que** ladite au moins une cavité (24) configurée pour mouler ledit cône fémoral (34) a une hauteur A₁ égale à 25mm, un plus petit diamètre C₁ choisi entre 16,08mm et 17,98mm et un plus grand diamètre D₁ choisi entre 27,12mm et 28,83mm.

6. Système (1) selon l'une quelconque des revendications précédentes, **caractérisé en ce que** ladite au moins une cavité (25) configurée pour mouler ledit cône fémoral (35) a une longueur maximale C₂ choisie entre 25,5mm et 27,42 mm et une largeur maximale D₂ choisie entre 22,58 mm et 24,28 mm.

7. Système (1) selon l'une quelconque des revendications précédentes, **caractérisé en ce que** la surface extérieure de ladite au moins une cavité (24) configurée pour mouler un cône fémoral (34) et de ladite au moins une cavité (25) configurée pour mouler un cône fémoral (35) comprend une partie avec des marches.

8. Procédé de fabrication d'au moins un élément compensatoire (31, 32, 33, 34, 35) au moyen d'un système (1) selon l'une des revendications 1-7, comprenant les étapes suivantes:
fourniture d'un système selon l'une quelconque des revendications 1-7;
- sélection d'une cavité (21, 22, 23, 24, 25) en fonction des aspects de forme et/ou de taille de l'élément compensatoire à produire;
- introduction de ciment osseux dans ladite cavité (21, 22, 23, 24, 25); et
- extraction de l'élément compensateur de ladite cavité (21, 22, 23, 24, 25) lorsque le ciment osseux a durci.

9. Procédé selon la revendication précédente, **caractérisé en ce qu'**il comprend en outre l'étape suivante
- presser le ciment osseux introduit dans ledit système (1).
